# EUROPÄISCHE PATENTSCHRIFT

(11) **EP 0 997 534 B1**
(45) Veröffentlichungstag und Bekanntmachung des Hinweises auf die Patenterteilung: **18.04.2007**
(21) Anmeldenummer: 99118777.4
(22) Anmeldetag: 18.09.1999
(51) Int. Cl.: C12P 41/00, C12N 9/20, C07C 69/01, C12P 7/62

(54) **Verfahren zur Racematspaltung von Arylalkylcarbonsäureestern**
Process for the resolution of racemic arylalkylcarboxylic acid esters
Procédé pour le dédoublement du mélange racémique d'esters arylalkyl d'acides carboxyliques

(30) Priorität: 30.09.1998 DE 19844876
(43) Veröffentlichungstag der Anmeldung: 03.05.2000
(73) Patentinhaber: BASF Aktiengesellschaft, 67056 Ludwigshafen (DE)
(72) Erfinder: Bornscheuer, Uwe, Dr., 70569 Stuttgart (DE); Henke, Erik, 70180 Stuttgart (DE); Hong, Yang, Dr., O'Connor ACT 2602, Canberra (AU)

(56) Entgegenhaltungen:
- EP-A- 0 402 771
- EP-A- 1 031 629
- HENKE, ERIK ET AL: "Lipase -catalyzed resolution of ibuprofen" MONATSHEFTE FUER CHEMIE, Bd. 131, Nr. 6, 15. Juni 2000 (2000-06-15), Seiten 633-638, XP001153340
- YAMAZAKI, YOSHIMITSU ET AL: "Diametric stereoselectivity of Pseudomonas fluorescens lipase and Candida cylindracea lipase in the acylation of organometallic alcohols" AGRICULTURAL AND BIOLOGICAL CHEMISTRY (1990), 54(12), 3357-61 , 1990, XP009013212
- MIYAZAWA, TOSHIFUMI ET AL: "Resolution of racemic carboxylic acids via the lipase -catalyzed irreversible transesterification of vinyl esters" CHIRALITY , Bd. 11, Nr. 7, 1999, Seiten 554-560, XP009013209
- YANG, HONG ET AL: "The Use of Vinyl Esters Significantly Enhanced Enantioselectivities and Reaction Rates in Lipase -Catalyzed Resolutions of Arylaliphatic Carboxylic Acids" JOURNAL OF ORGANIC CHEMISTRY, Bd. 64, Nr. 5, 5. März 1999 (1999-03-05), Seiten 1709-1712, XP002246387
- YANG, HONG ET AL: "Highly efficient double enantioselection by lipase -catalyzed transesterification of (R,S)-carboxylic acid vinyl esters with (RS)-1-phenylethanol" TETRAHEDRON: ASYMMETRY, Bd. 10, 12. März 1999 (1999-03-12), Seiten 957-960, XP004162519

## Beschreibung

Die Erfindung betrifft ein Verfahren zur Racematspaltung von Arylalkylcarbonsäureestern.

Lipasen und Esterasen zeichnen sich durch ein breites Substratspektrum verbunden mit häufig sehr hoher Stereoselektivität und Stabilität auch in nicht-wäßrigen Lösungsmitteln aus. Die erfolgreiche Anwendung dieser Enzyme ist in einer Reihe umfangreicher Übersichtsartikel (Schoffers et al., Tetrahedron, 52, 1996: 3769 - 3826; Kazlauskas & Bornscheuer, Biotransformations, VCH, Weinheim, Vol. 8a, 1998: 37; Schmid et al., Angew. Chem. Int. Ed. Engl., 37, 1998: 1608) und in Büchern (F. Theil, Enzyme in der Organischen Synthese, Spektrum Akademischer Verlag, Heidelberg, 1997; C.-H. Wong, G.M. Whitesides, Enzymes in Synthetic Organic Chemistry, Pergamon Press, Oxford, 1994; K. Faber, Biotransformations in Organic Chemistry, A Textbook, 3 Edition, Springer, Berlin 1997; K. Drauz, H. Waldmann, Enzyme Catalysis in Organic Synthesis, A Comprehensive Handbook, Vol I + II, VCH, Weinheim, 1995) dokumentiert.

Über diese Enzymkatalyse sind optisch reine Alkohole gut zugänglich. Zur Darstellung dieser optisch reinen Alkohole wurden Umesterungen in organischem Lösungsmittel ausgehend von racemischen oder prostereogenen Vorstufen vielfach beschrieben. Zur Erhöhung der Reaktionsrate und zur Verschiebung des Reaktionsgleichgewichtes hat es sich als sehr nützlich erwiesen, aktivierte Ester, insbesondere Enolester, einzusetzen. Häufig eingesetzte Acyldonoren sind Vinylester und hierunter vor allem Vinylacetat. Der intermediär in der Umesterung gebildete Vinylalkohol lagert sich in einer Keto-Enol-Tautomerie in den leicht-flüchtigen Acetaldehyd um, was zu einer Unterdrückung der unerwünschten Rückreaktion führt (siehe Degueil-Castaing et al., Tetrahedron Lett., 28, 1987: 953 - 954; Wang et al., J. Am. Chem. Soc., 110, 1988: 7200 - 7205; Laumen et al., J. Chem. Soc., Chem. Commun., 1988: 1459 - 1461).

Anders sieht die Zugänglichkeit bei optisch aktiven Carbonsäuren aus. Im Gegensatz zur oben beschriebenen Darstellung von optisch reinen Alkoholen unter Ausnutzung der Syntheserichtung von Lipasen oder Esterasen in nicht-wäßrigen Lösungsmittel werden optisch reine Carbonsäuren in der Hydrolyserichtung, in der die entsprechenden Ester hydrolysiert werden, dargestellt. Dies ist notwendig, da die verwendeten Enzyme in der Syntheserichtung auf der Carbonsäureseite nur ein sehr geringes Substratspektrum akzeptieren und die Hydrolyserichtung ein weiteres Substratspektrum ermöglicht. Von Nachteil ist hierbei jedoch, daß wasserlabile Verbindungen unter diesen Bedingungen nicht verwendet werden können. Außerdem kann es im wäßrigen Milieu zu einer teilweisen oder vollständigen Racemisierung der Produkte. Dies führt zu einer Abnahme der optischen Reinheit der Produkte.

Die Ausnutzung der Syntheserichtung bei der Herstellung von chiralen Carbonsäuren mit nicht-aktivierten Estern ist ebenfalls literaturbekannt (siehe Holmberg et al., Appl. Microbiol. Biotechnol., 35, 1992: 572 - 578; Persichetti et al., Tetrahedron Lett., 37, 1996: 6507 - 6510; Ozegowski et al., Liebigs Ann. 1994: 215 - 217). Sie ist jedoch neben dem oben schon erwähnten Nachteil der geringen Substratbreite mit weiteren Nachteilen behaftet. Die Gleichgewichtslage bei der Racematspaltung in einer Umesterungsreaktion mit nicht-aktivierten Estern liegt sehr ungünstig und führt entweder zu Produktgemischen oder zu sehr langen Reaktionszeiten. Um diese Nachteile zu überwinden kann die Reaktion zur Verschiebung des Gleichgewichts in Gegenwart eines Molekularsiebs (Soumanou et al., J. Am. Oil Chem. Soc., 75, 1998: 703 - 710), bei erniedrigtem Druck oder unter Anlegung eines Vakuums (Björkling et al., J. Chem. Soc., Chem. Commun., 1989: 934 - 935) oder als Festphasensythese (McNeill et al., J. Am. Oil Chem. Soc., 67, 1990: 779 - 783; Cao et al., Biocatal. Biotransform., 14, 1997: 269 - 283) durchgeführt werden, was jedoch in jedem Fall zu einer technisch aufwendigeren Reaktion führt.

Yamazaki et al., Agricultural and Biological Chemistry, 54(12), 1990: 3357-3361 offenbart ein Verfahren zur Racematspaltung von 2-Phenylpropionsäurevinylestern durch Umesterung in Gegenwart eines aromatischen Alkohols (Benzylalkohol-tricarbonylchromium).

Es war daher die Aufgabe der Erfindung ein technischen Zugang optisch aktiven Carbonsäuren zu entwickeln, das die oben genannten Nachteile nicht aufweist und leicht durchführbar ist.

Diese Aufgabe wurde durch das erfindungsgemäße Verfahren zur Racematspaltung von Arylalkylcarbonsäureestern der allgemeinen Formel I 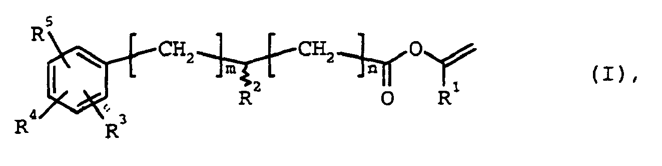 dadurch gekennzeichnet, daß man Verbindungen der allgemeinen Formel I in Gegenwart einer Lipase oder Esterase mit einem Alkohol der allgemeinen Formel R⁶-OH zu Verbindungen der allgemeinen Formeln Ia und II 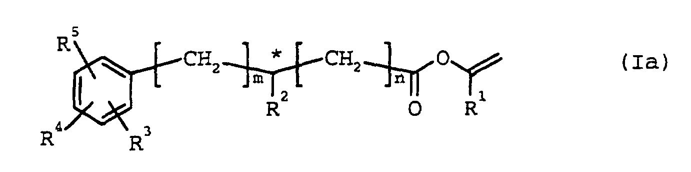 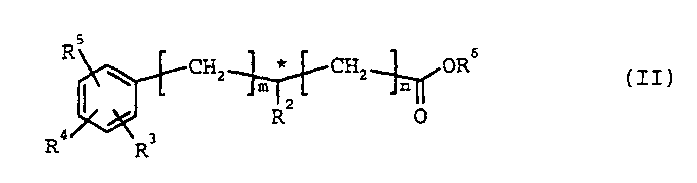 umsetzt, wobei mindestens eine der Verbindungen der Formeln Ia oder II in einem Enantiomerenüberschuß vorliegt und die Substituenten und Variablen in den Formeln I, Ia und II folgende Bedeutung haben:
- * =: optisch aktives Zentrum
- n und m: unabhängig voneinander 0 oder 1
- R¹ =: Wasserstoff oder Methyl
- R²: = substituiertes oder unsubstituiertes, verzweigtes oder unverzweigtes C₁-C₆-Alkyl-, C₃-C₆-Cycloalkyl-, Aryl- oder Hetaryl-,
- R³, R⁴, R⁵: unabhängig voneinander Wasserstoff, substituiertes oder unsubstituiertes, verzweigtes oder unverzweigtes C₁-C₁₀-Alkyl-, C₂-C₁₀-Alkenyl-, C₂-C₁₀-Alkinyl-, C₁-C₁₀-Alkoxy-, C₂-C₁₀-Alkenyloxy-, C₂-C₁₀-Alkinyloxy-, C₃-C₁₀-Cycloalkyl-, C₃-C₁₀-Cycloalkyloxy-, C₁-C₄-Alkylaryl-, C₁-C₄-Alkylhetaryl-, Aryl-, Hetaryl-, Hydroxyl-, Halogen-, Cyano-, Nitro- oder Amino-,
- R⁶ =: substituiertes oder unsubstituiertes, verzweigtes oder unverzweigtes C₁-C₂₀-Alkyl-, C₁-C₂₀-Alkoxy-, C₂-C₂₀-Alkenyl- oder C₃-C₁₀-Cycloalkyl-,
und wobei zwei benachbarte Substituenten R³, R⁴ oder R⁵ zusammen einen weiteren substituierten oder unsubstituierten aromatischen, gesättigten oder teilweise gesättigten Ring mit 5 bis 6 Atomen im Ring bilden können, der ein oder mehrere Heteroatome wie O, N oder S enthalten kann, gelöst.

R² bezeichnet in den Verbindungen der Formeln I, Ia und II substituiertes oder unsubstituiertes, verzweigtes oder unverzweigtes C₁-C₆-Alkyl-, C₃-C₆-Cycloalkyl-, Aryl- oder Hetaryl-.

Als Alkylreste seien substituierte oder unsubstituierte verzweigte oder unverzweigte C₁-C₆-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl genannt. Bevorzugt sind Methyl, Ethyl, n-Propyl, n-Butyl, i-Propyl oder i-Butyl.

Als Cycloalkylreste in der Formel seien beispielhaft substituierte oder unsubstituierte verzweigte oder unverzweigte C₃-C₆-Cycloalkylketten mit 3 bis 6 Kohlenstoffatomen im Ring oder Ringsystem wie Cyclopropyl, Cyclobutyl, Cyclopentyl, Cyclohexyl, 1-Methylcyclopropyl, 1-Ethylcyclopropyl oder 1-Propylcyclopropyl genannt. Die Cycloalkylreste können auch Heteroatome wie S, N und O im Ring enthalten.

Als Aryl- seihen substituiertes und unsubstituiertes Phenyl oder Naphtyl genannt. Bevorzugt sind Phenyl oder Naphtyl.

Als Hetarylreste seien substituierte und unsubstituierte Hetarylreste, die ein oder mehrere Stickstoff-, Schwefel- und/oder Sauerstoffatome im Ring oder Ringsystem enthalten, genannt.

Als Substituenten der genannten Reste von R² kommen beispielsweise ein oder mehrere Substituenten wie Halogen wie Fluor, Chlor oder Brom, Cyano, Nitro, Amino oder Hydroxy in Frage. Bevorzugt sind Methyl, Chlor oder Hydroxy.

R³, R⁴, R⁵ bezeichnet in den Verbindungen der Formeln I, Ia und II unabhängig voneinander Wasserstoff, substituiertes oder unsubstituiertes, verzweigtes oder unverzweigtes C₁-C₁₀-Alkyl-, C₂-C₁₀-Alkenyl-, C₂-C₁₀-Alkinyl-, C₁-C₁₀-Alkoxy-, C₂-C₁₀-Alkenyloxy-, C₂-C₁₀-Alkinyloxy-, C₃-C₁₀-Cycloalkyl-, C₃-C₁₀-Cycloalkyloxy-, C₁-C₄-Alkylaryl-, C₁-C₄-Alkylhetaryl-, Aryl-, Hetaryl-, Hydroxyl-, Halogen- wie Fluor, Chlor oder Brom, Cyano-, Nitro- oder Amino-. Weiter können zwei benachbarte Substituenten R³, R⁴ oder R⁵ zusammen einen weiteren substituierten oder unsubstituierten aromatischen, gesättigten oder teilweise gesättigten Ring mit 5 bis 6 Atomen im Ring bilden, der ein oder mehrere Heteroatome wie O, N oder S enthalten kann; so das kondensierte Systeme entstehen, wobei nicht mehr als ein Ring an den zentralen Ring ankondensiert sein kann.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylperityl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl, 1-Ethyl-2-methylpropyl, n-Heptyl, n-Octyl, n-Nonyl oder n-Decyl genannt. Bevorzugt sind Methyl, Ethyl, n-Propyl, n-Butyl, i-Propyl oder i-Butyl.

Als Alkenylreste seien substituierte oder unsubstituierte, verzweigte oder unverzweigte C₂-C₁₀-Alkenylketten, wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, 1-Nonenyl, 2-Nonenyl, 3-Nonenyl, 4-Nonenyl, 5-Nonenyl, 6-Nonenyl, 7-Nonenyl, 8-Nonenyl, 1-Decenyl, 2-Decenyl, 3-Decenyl, 4-Decenyl, 5-Decenyl, 6-Decenyl, 7-Decenyl, 8-Decenyl oder 9-Decenyl genannt.

Als Alkinylreste seien substituierte oder unsubstituierte, verzweigte oder unverzweigte C₂-C₁₀-Alkinylketten, wie beispielsweise Ethinyl-, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl und die höheren Homologen dieser Reihe genannt.

Als Alkoxyreste seien substituierte oder unsubstituierte, verzweigte oder unverzweigte C₁-C₁₀-Alkoxyketten wie beispielsweise Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, Hexyloxy, Heptyloxy, Octyloxy, Nonyloxy oder Decyloxy und deren verzweigtkettige Homologen genannt.

Als Alkenyloxyreste seien substituierte oder unsubstituierte, verzweigte oder unverzweigte C₂-C₁₀-Alkenyloxyketten, wie beispielsweise Ethenyloxy, Propenyloxy, 1-Butenyloxy, 2-Butenyloxy, 3-Butenyloxy, 2-Methylpropenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-1-butenyloxy, 1,2-Dimethyl-2-butenyloxy, 1,2-Dimethyl-3-butenyloxy, 1,3-Di-methyl-1-butenyloxy, 1,3-Dimethyl-2-butenyloxy, 1,3-Dimethyl-3-butenyloxy, 2,2-Dimethyl-3-butenyoxyl, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy, 1-Ethyl-2-methyl-2-propenyloxy, 1-Heptenyloxy, 2-Heptenyloxy, 3-Heptenyloxy, 4-Heptenyloxy, 5-Heptenyloxy, 6-Heptenyloxy, 1-Octenyloxy, 2-Octenyloxy, 3-Octenyloxy, 4-Octenyloxy, 5-Octenyloxy, 6-Octenyloxy, 7-Octenyloxy, 1-Nonenyloxy, 2-Nonenyloxy, 3-Nonenyloxy, 4-Nonenyloxy, 5-Nonenyloxy, 6-Nonenyloxy, 7-Nonenyloxy, 8-Nonenyloxy, 1-Decenyloxy, 2-Decenyloxy, 3-Decenyloxy, 4-Decenyloxy, 5-Decenyloxy, 6-Decenyloxy, 7-Decenyloxy, 8-Decenyloxy oder 9-Decenyloxy genannt.

Als Alkinyloxyreste seien substituierte oder unsubstituierte, verzweigte oder unverzweigte C₂-C₁₀-Alkinyloxyketten, wie beispielsweise Ethinyloxy-, Prop-1-in-1-yloxy, Prop-2-in-1-yloxy, n-But-1-in-1-yloxy, n-But-1-in-3-yloxy, n-But-1-in-4-yloxy, n-But-2-in-1-yloxy, n-Pent-1-in-1-yloxy, n-Pent-1-in-3-yloxy, n-Pent-1-in-4-yloxy, n-Pent-1-in-5-yloxy, n-Pent-2-in-1-yloxy, n-Pent-2-in-4-yloxy, n-Pent-2-in-5-yloxy, 3-Methyl-but-1-in-3-yloxy, 3-Methyl-but-1-in-4-yloxy, n-Hex-1-in-1-yloxy, n-Hex-1-in-3-yloxy, n-Hex-1-in-4-yloxy, n-Hex-1-in-5-yloxy, n-Hex-1-in-6-yloxy, n-Hex-2-in-1-yloxy, n-Hex-2-in-4-yloxy, n-Hex-2-in-5-yl, n-Hex-2-in-6-yloxy, n-Hex-3-in-1-yloxy, n-Hex-3-in-2-yloxy, 3-Methyl-pent-1-in-1-yloxy, 3-Methyl-pent-1-in-3-yloxy, 3-Methyl-pent-1-in-4-yloxy, 3-Methyl-pent-1-in-5-yloxy, 4-Methyl-pent-1-in-1-yloxy, 4-Methyl-pent-2-in-4-yloxy oder 4-Methyl-pent-2-in-5-yloxy und die höheren Homologen dieser Reihe genannt.

Als Cycloalkylreste seien beispielhaft substituierte oder unsubstituierte verzweigte oder unverzweigte C₃-C₁₀-Cycloalkylketten mit 3 bis 7 Kohlenstoffatomen im Ring oder Ringsystem wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt. Die Cycloalkylreste können auch Heteroatome wie S, N und O im Ring enthalten.

Als Cycloalkyloxyreste seien beispielhaft substituierte oder unsubstituierte verzweigte oder unverzweigte C₃-C₁₀-Cycloalkyloxyketten mit 3 bis 7 Kohlenstoffatomen im Ring oder Ringsystem wie Cyclopropyloxy, Cyclobutyloxy, Cyclopentyloxy, Cyclohexyloxy, Cycloheptyloxy, 1-Methylcyclopropyloxy, 1-Ethylcyclopropyloxy, 1-Propylcyclopropyloxy, 1-Butylcyclopropyloxy, 1-Pentylcyclopropyloxy, 1-Methyl-1-Butylcyclopropyloxy, 1,2-Dimethylcyclypropyloxy, 1-Methyl-2-Ethylcyclopropyloxy, Cyclooctyloxy, Cyclononyloxy oder Cyclodecyloxy genannt. Die Cycloalkyloxyreste können auch Heteroatome wie S, N und O im Ring enthalten.

Als C₁-C₄-Alkylaryl seien substituierte und unsubstituierte verzweigtkettige oder unverzweigtkettige C₁-C₄-Alkyl-phenyl- oder C₁-C₄-Alkyl-naphthylreste wie Methylphenyl, Ethylphenyl, Propylphenyl, 1-Methylethylphenyl, Butylphenyl, 1-Methylpropylphenyl, 2-Methylpropylphenyl, 1,1-Dimethylethylphenyl, Methylnaphthyl, Ethylnaphthyl, Propynaphthyl, 1-Methylethylnaphthyl, Butylnaphthyl, 1-Methylpropylnaphthyl, 2-Methylpropylnaphthyl oder 1,1-Dimethylethylnaphthyl genannt.

Als Alkylhetarylreste seien substituierte und unsubstituierte verzweigtkettige oder unverzweigtkettige C₁-C₄-Alkylhetarylreste, die ein oder mehrere Stickstoff-, Schwefel- und/oder Sauerstoffatome im Ring oder Ringsystem enthalten, genannt.

Unter Aryl sind beispielsweise einfache oder kondensierte aromatische Ringsysteme, die ggf. mit einem oder mehreren Resten wie Halogen wie Fluor, Chlor oder Brom, Cyano, Nitro, Amino, Hydroxy, Thio, Alkyl, Alkoxy, Aryl, Hetaryl oder weiteren gesättigten oder ungesättigten nicht aromatischen Ringen oder Ringsystemen substituiert sein können, zu verstehen. Bevorzugt sind ggf. substituierte Phenyl, Methoxyphenyl und Naphthyl.

Unter Hetaryl sind beispielsweise einfache oder kondensierte aromatische Ringsysteme mit einem oder mehreren heteroaromatischen 3- bis 7gliedrigen Ringen, die ein oder mehrere Heteroatome wie N, O oder S enthalten können, und die ggf. mit einem oder mehreren Resten wie Halogen wie Fluor, Chlor oder Brom, Cyano, Nitro, Amino, Hydroxy, Thio, Alkyl, Alkoxy oder weiteren aromatischen oder weiteren gesättigten oder ungesättigten nicht aromatischen Ringen oder Ringsystemen substituiert sein können, zu verstehen.

Als Substituenten der genannten Reste von R^{3,} R⁴ und R⁵ kommen prinzipiell alle denkbaren Substituenten in Frage beispielsweise ein oder mehrere Substituenten wie Halogen wie Fluor, Chlor oder Brom, Cyano, Nitro, Amino, Hydroxy, Alkyl, Cycloalkyl, Aryl, Alkoxy, Benzyloxy, Phenyl oder Benzyl.

R⁶ bezeichnet in den Verbindungen der Formeln II unabhängig substituiertes oder unsubstituiertes, verzweigtes oder unverzweigtes C₁-C₂₀-Alkyl-, C₁-C₂₀-Alkoxy-, C₂-C₂₀-Alkenyl-, oder C₃-C₁₀-Cycloalkyl-.

Als Alkylreste seien substituierte oder unsubstituierte, verzweigte oder unverzweigte C₁-C₂₀-Alkylketten, wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosanyl genannt.

Als Alkyloxyreste seien substituierte oder unsubstituierte, verzweigte oder unverzweigte C₁-C₂₀-Alkyloxyketten, wie beispielsweise Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, Hexyloxy, Heptyloxy, Octyloxy, Nonyloxy, Decyloxy, Undecyloxy, Dodecyloxy, Tridecyloxy, Tetradecyloxy, Pentadecyloxy, Hexadecyloxy, Heptadecyloxy, Octadecyloxy, Nonadecyloxy oder Eicosanyloxy genannt.

Als Alkenylreste seien substituierte oder unsubstituierte, verzweigte oder unverzweigte C₂-C₂₀-Alkenylketten, wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, 1-Nonenyl, 2-Nonenyl, 3-Nonenyl, 4-Nonenyl, 5-Nonenyl, 6-Nonenyl, 7-Nonenyl, 8-Nonenyl, 1-Decenyl, 2-Decenyl, 3-Decenyl, 4-Decenyl, 5-Decenyl, 6-Decenyl, 7-Decenyl, 8-Decenyl oder 9-Decenyl und deren höhere Homologe genannt.

Als Cycloalkylreste seien beispielhaft substituierte oder unsubstituierte verzweigte oder unverzweigte C₃-C₁₀-Cycloalkylketten mit 3 bis 7 Kohlenstoffatomen im Ring oder Ringsystem wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt. Die Cycloalkylreste können auch Heteroatome wie S, N und O im Ring enthalten.

Das erfindungsgemäße Verfahren unter Verwendung der Vinylester oder der alkylierten Vinylester (wie Isopropenylester) führt zu einer vorteilhaften Steigerung der Reaktionsgeschwindigkeit bei gleichzeitiger erheblicher Steigerung der Enantioselektivität der verwendeten Enzyme (Lipasen und Esterasen). Es lassen sich so Enantiomerenüberschüsse bei hohen Reaktionsumsätzen erzielen, die weder in der Hydrolyserichtung noch bei der Umesterung mit nicht aktivierten Estern erreichbar sind. Es werden bei ca. 40 % Umsatz Enantiomerenreinheiten für das Substrat von mindestens 60 % ee, bevorzugt mindestens 70 %ee erreicht. Die Enantiomerenreinheiten für das Produkt liegt vorteilhaft bei mindestens 30 %ee, bevorzugt bei 50 %ee. Die Reaktionsgeschwindigkeit kann um mindestens das 10fache gegenüber den nicht-aktivierten Estern gesteigert werden, bevorzugt mindestens um das 20fache, besonders bevorzugt mindestens um das 30fache gesteigert werden. Die Umsetzung in beispielsweise Phosphatpuffer ist deutlich höher, jedoch ist die Selektivität unakzeptabel. Unter den erfindungsgemäßen Bedingungen lassen sich Enantioselektivitäten (= E) von mindestens E = 20 bis zu E > 100 erreichen (Berechnung siehe Beispiele).

Das erfindungsgemäße Verfahren wird vorteilhaft in Gegenwart von mindestens einem weiteren organischen Lösungsmittel durchgeführt, kann aber auch ohne Anwesenheit weiterer Lösungsmittel durchgeführt werden. In diesem Fall dient der für die Umesterung verwendete Alkohol (R⁶-OH) als Lösungsmittel. Als Alkohole können prinzipiell alle primären und sekundären Alkohole verwendet werden. Sekundäre Alkohole zeigen in der Reaktion jedoch eine geringere Aktivität, tertiäre Alkohole können im Prinzip auch verwendet werden. Die Reaktionszeiten mit diesen Alkoholen sind jedoch unbefriedigend. Als Beispiele für vorteilhafte Alkohole seien primäre aliphatische verzweigte oder unverzweigte Alkohole wie Methanol, Ethanol, Propanol, Butanol, Pentanol, Hexanol, Heptanol, Nonanol oder Decanol genannt. Als sekundäre Alkohole seien aliphatische Alkohole wie i-Propanol oder i-Butanol genannt. Auch cyklische aliphatische Alkohole oder aromatische Alkohole können verwendet werden. Bevorzugt werden Methanol, Ethanol, Isopropanol oder Hexanol verwendet.

Als zusätzliche Lösungsmittel kommen alle organischen Lösungsmittel in Frage, die die Edukt- und Produktlöslichkeit steigern können und so die Reaktionszeit positiv beeinflussen. Vorteilhaft werden aprotische Lösungsmittel wie Toluol, Hexan oder Benzol oder polar aprotische Lösungsmittel wie DMSO, DMF oder N-Methylpyrrolidon für das erfindungsgemäße Verfahren verwendet.

Das erfindungsgemäße Verfahren läßt sich bei Temperaturen zwischen -50 °C und +100 °C durchführen. Bei Verwendung thermostabiler Enzyme können auch höhere Umsatztemperaturen erreicht werden (siehe beispielsweise Ikeda et al, Molecular cloning of extremely thermostable esterase gene from hyperthermophilic archaeon Pyrococcus furiosus in Escherichia coli, Biotechnol. Bioeng., 57, 1998: 624 - 629). Im Bereich von 0 °C oder darunter nimmt die Reaktionsgeschwindigkeit deutlich ab. Eine Reaktion in diesem Bereich ist jedoch prinzipiell möglich wie Sakai et al. zu entnehmen ist (Enhancement of the enantioselectivity in lipasecatalysed kinetic resolutions of 3-phenyl-2H-azirine-2-methanol by lowering the temperature to -40 degree, J. Org. Chem., 62, 1997: 4906 - 4907). Bevorzugt wird das Verfahren zwischen 0 °C und 90 °C, besonders bevorzugt zwischen 10 °C und 80 °C durchgeführt.

Für das erfindungsgemäße Verfahren sind prinzipiell alle Lipasen oder Esterasen wie mikrobielle, tierische oder pflanzliche Lipasen oder Esterasen geeignet. Vorteilhaft werden bakterielle oder pilzliche Lipasen oder Esterasen oder Schweinepankreaslipase verwendet. Bevorzugt werden Lipasen verwendet. Als Lipasen sind bakterielle oder pilzliche Lipasen geeignet, die aus den Gattungen Arthrobacter, Alcaligenes, Bacillus, Brevibacterium, Pseudomonas, Chromobacterium, Aspergillus, Candida, Fusarium, Geotrichum, Humicola, Mucor, Pichia oder Rhizopus isoliert wurden. Die Reaktion kann auch in Gegenwart der gesamten Organismen oder Rohextrakten der Organismen durchgeführt werden. Vorteilhaft geeignet sind Lipasen aus den Gattungen und Arten Arthrobacter sp., Alcaligenes sp., Bacillus cereus, Bacillus subtilis, Bacillus coagulans, Brevibacterium ammoniagenes, Pseudomonas aeruginosa, Pseudomonas cepacia, Pseudomonas fluorescens, Pseudomonas sp., Chromobacterium viscosum, Aspergillus niger, Candida antartica, Candida cylindracea, Candida rugosa, Fusarium solani, Geotrichum candidum, Humicola lanuginosa, Mucor javonicus, Mucor mihei, Mucor sp., Pichia miso, Rhizopus nigricans, Rhizopus oryzae oder Rhizopus sp.. Auch kommerziell erhältliche Lipasen oder Formulierungen dieser Lipasen, die beispielsweise von den Firmen Amano, Novo oder Boehringer Mannheim erhältlich sind, sind für das erfindungsgemäße Verfahren geeignet. Bevorzugt sind die Lipasen aus Candida antartica, die in zwei Isoformen A oder B oder deren Gemisch erhältlich ist, oder Lipasen aus Candida cylindracea. Diese Enzyme eignen sich als freie Enzyme oder als Enzymformulierungen beispielsweise als Lipase Chirazyme L2 von der Firma Boehringer Mannheim oder als Novozym 435 der Firma Novo für das erfindungsgemäße Verfahren. Besonders bevorzugt ist die Candida antartica Lipase B (= CAL-B).

Vorteilhaft wird das erfindungsgemäße Verfahren in einer Kombination von primären Alkohol mit einem aprotischen Lösungsmittel durchgeführt, wie der Kombination aus n-Hexanol und Toluol. Das Edukt (= racemischer Vinylester oder racemischer alkylierter Vinylester) wird im Alkohol oder im Lösungsmittel/Alkoholgemisch vorgelegt. Die Reaktion wird durch Zugabe des Enzyms gestartet. Schema I zeigt beispielhaft eine entsprechende Reaktion. Als Produkte entstehen der Vinylester, der Hexylester und in Schema I nicht dargestellt der Aldehyd oder das Keton. Mindestens eines der Reaktionsprodukte Vinylester (= Substrat) oder Hexylester (= Produkt, siehe oben) zeigen in der Reaktion einen Enantiomerenüberschuß. Bevorzugt werden Enantiomerenreinheiten wie oben beschrieben erreicht.

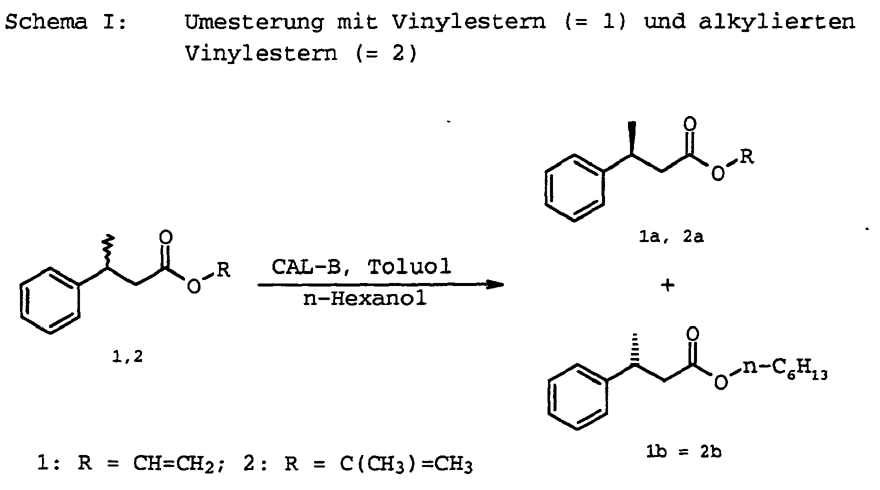

Mit dem erfindungsgemäßen Verfahren lassen sich vorteilhaft rasch optisch aktive Carbonsäuren in guten Ausbeuten (> 40 % Ausbeute) und hohen Enantiomerenreinheiten (von mindestens 60 % ee) herstellen.

### Beispiele

Wenn nicht anders beschrieben wurden die ¹H-NMR-Spektren bei 250.1 und 500.1 MHz und die ¹³C-NMR-Spektren bei 62.9 und 125.7 MHz in CDCl₃ mit Tetramethylsilan als internem Standard aufgenommen. In Fällen in denen sowohl das Racemat als auch die enantiomeren reinen Verbindungen synthetisiert wurden, wurde das Racemat zur vollen Charakterisierung der Verbindung verwendet. Chemische und optische Reinheit der Verbindungen wurden über NMR- und GC-Analyse im Vergleich zum Racemat nachgewiesen bzw. bestimmt. Die Gas-Chromatographie- (= GC-)Analysen wurden mit einer Optima 5-Säule (25 m x 0,25 mm; Macherey & Nagel, Düren, Germany) zur Bestimmung des Umsatzes und Reinheit und mit einer Heptakis-(2,3-di-O-acetyl-6-O-TBDMS)-β-cyclodextrin-Säule (25 m x 0,25 mm, Prof. W.A. König, Universität Hamburg, Germany) zur Bestimmung des Enantiomerenüberschusses durchgeführt. Als immobilisierte Lipase wurde Candida antarctica Lipase (Chiracyme L-2, c.-f., C2, 5000 U/g) der Firma Boehringer Mannheim, Penzberg, Germany, wenn nicht anders beschrieben, verwendet. Die absolute Konfiguration der Biotransformationsprodukte wurde über Vergleich mit optisch reinen Standards, die aus den kommerziell erhältlichen optisch reinen Carbonsäuren hergestellt wurden, bestimmt. Der Enantiomerenüberschuß wurde durch Vergleich mit Literaturwerten berechnet. Die Enantioselektivität wurde mit der Formel E = [ln{(1-c) x (1-eeₛ)}]/[ln{(1-c) x (1+eeₛ)}], bestimmt.

### Herstellung der Vinylester und Analysen

Die Vinylester wurden nach der von Wang et al. (J. Am. Chem. Soc., 110, 1988: 7200) beschriebenen Methode hergestellt. 500 mg der Carbonsäuren und HgOAc₂ (70 mg, 0,22 mmol)wurden in 10 ml Vinylacetat gelöst. Die Lösung wurden 30 Min. bei Raumtemperatur (= ca. 23 °C) gerührt, anschließend wurden 0,1 ml konz. H₂SO₄ zugegeben. Die Lösung 6 Stunden refluxiert und auf Raumtemperatur abgekühlt. Danach wurden 400 mg NaOAc zum Quenchen des Katalysators zugegeben. Die Lösung wurde gefiltert und aufkonzentriert. Die Rohprodukte wurde anschließend über Silicagelchromatographie (Petrolether:Et₂O, 20:1) aufgereinigt. Alle Vinylester wurden als farblose Flüssigkeiten erhalten, die keiner weiteren Aufreinigung bedurften. Optisch reine Vinylester wurden in kleineren Mengen unter Verwendung von 60 - 120 mg optisch reiner Carbonsäuren synthetisiert.

(±)-2-Phenylbuttersäurevinylester (±)-3: Die Herstellung erfolgte nach der oben beschriebenen Methode unter Verwendung von 500 mg (= 3.05 mmol) der entsprechenden Carbonsäure [2-Phenylbuttersäure = (±)-12]. Es wurden 290 mg (±)-3 (1,52 mmol, 50 %) erhalten. Analysen Ergebnis: C, 75.80; H, 7.41.; kalk. für C₁₂H₁₄O₂: C, 75.76; H, 7.42; ¹H NMR (500.1 MHz; CDCl₃) δ 0.91 (t, J = 7.4, 3H), 1.80-2.16 (m, 2H), 3.51 (t, J = 7.7, 1H), 4.54 (dd, J = 6.32, 1.6, 1H), 4.85 (dd, J = 14.0, 1.7, 1H), 7.23-7.34 (m, 6H); ¹³C NMR (125.8 MHz; CDCl₃) δ 12.08, 26.62, 53.17, 97.90, 127.41, 128.00, 128.67, 138.28, 141.32, 171.12, IR (KBr)/cm⁻¹: 3080w, 3050w, 3015w, 2955vs, 2920s, 2860w, 1750vs, 1640vs, 1595w, 1475s, 1447s, 1130br, 860s, 720s, 680vs.

R-(-)-2-Phenylbuttersäurevinylester R-(-)-3: 120 µl R-(-)-12 (126.6 mg, 0.77 mmol) erbrachten 44 mg R-(-)-3 (0.23 mmol, 30 %); [α]_{D}²² = -23.9 (c0.664, CHCl₃).

(±)-2-Phenylpropionsäurevinylester (±)-4: 420 µl (±)-13 (460.7 mg, 3.07 mmol) erbrachten 210 mg (±)-4 (1.19 mmol, 39 %): Analysen Ergebnis: C, 74.73; H, 6.93; kalk. für C₁₁H₁₂O₂; C, 74.98; H, 6.86; ¹H NMR (500.1 MHz; CDCl₃) 1.53 (d, J = 7.2, 3H), 3.79 (q, J = 7.1, 1H), 4.54 (d, J = 6.18; 1H), 4.77 (d, J = 14.0, 1H), 7.23-7.35 (m, 6H); ¹³C NMR (125.7 MHz; CDCl₃) 18.41, 45.26, 97.92, 127.36, 127.52, 128,73, 139.71, 141.36, 171.59; IR (KBr)/cm⁻¹: 3080w, 3050w, 3020s, 2970s, 2920s, 2860w, 1750vs, 1640vs, 1595w, 1485s, 1445s, 1140br, 860s, 715s, 680vs.

R-(-)-2-Phenylpropionsäurevinylester R-(-)-4: 60 µl R-(-)-13 (65.8 mg, 0.44 mmol) erbrachten 18 mg R-(-)-4 (0.10 mmol, 23 %) [α]_{D}²² = -34.6 (c0.900, EtOH).

(±)-3-Phenylbuttersäurevinylester (±)-5: 500 mg (±)-14 (3.05 mmol) erbrachten 300 mg (±)-5 (1.58 mmol, 52 %): Analysen Ergebnis: C, 75.63; H, 7.68; kalk. für C₁₂H₁₄O₂: C, 75.76; H, 7.42; ¹H NMR (250.1 MHz; CDCl₃) δ 1.25 (d, J = 7.0, 3H), 2.59 (m, 2H), 3.24 (m, 1H), 4.47 (dd, J = 6.3, 1.5, 1H), 4.77 (dd, J = 14.0, 1.5, 1H), 7.13-7.27 (m, 6H); ¹³C NMR (62.9 MHz; CDCl₃) δ 21.82, 36.27, 42.60, 97.76, 126.63, 126.78, 128.66, 141.18, 145.41, 169.48; IR (KBr)/cm⁻¹: 3070w, 3050w, 3010s, 2950s, 2910w, 2860w, 1750vs, 1640vs, 1595w, 1485s, 1445s, 1140br, 860s, 745s, 680vs.

R-(-)-3-Phenylbuttersäurevinylester R-(-)-5: 120 µl R-(-)-14 (128.3 mg, 0.78 mmol) erbrachten 29 mg R-(-)-5 (0.15 mmol, 19 %) [α]_{D}²² = -21.2 (c1.543, 1,4-Dioxan).

### Herstellung der Ethylester und Analysen

Zur Herstellung der Ethylester wurden die entsprechenden Carbonsäuren in einem Gemisch aus EtOH (= Ethanol, 15 ml) und Toluol (100 ml) in einem 250 ml Zweihalskolben mit Rückflußkühler und Dean-Stark-Falle unter Rühren gelöst. Anschließend wurden 0,5 ml konz. H₂SO₄ zugegeben. Das Reaktionsgemisch wurde unter Rückfluß erhitzt, bis sich kein Wasser mehr abscheidet (ca. 6 Std.). Nach Abkühlung auf Raumtemperatur (ca. 23 °C) wurde das Reaktionsgemisch in einem Scheidetrichter mit 100 ml Eiswasser, 100 ml gesättigter Natriumhydrogencarbonatlösung und wieder 100 ml Eiswasser gewaschen. Die organische Phase wurde über Natriumsulfat getrocknet, filtriert und über Silicagel-Chromatographie (Petrolether:Ether, 15:1) wurde das Produkt gereinigt. Die Ethylester wurden als farblose Flüssigkeit (3,1 g, 53 %) isoliert. Optisch reine Ethylester wurden in kleineren Mengen unter Verwendung von 60 - 120 mg Carbonsäure synthetisiert.

(±)-2-Phenylbuttersäureethylester (±)-6: Die Herstellung erfolgte nach der oben beschriebenen Methode unter Verwendung von 5.0 g (= 30.5 mmol) der entsprechenden Carbonsäure [2-Phenylbutersäure = (±)-12]. Es wurden 3.1 g (±)-6 (16.1 mmol, 53 %) als farbloses Öl erhalten. Analysen Ergebnis: C, 74.96; H, 8.54; kalk. für C₁₂H₁₆O₂; C, 74.97; H, 8.39; ¹H NMR (250.1 MHz; CDCl₃) δ 0.82 (t, J = 7.4, 3H), 1.13 (t, J = 7.1, 3H), 1.66-2.08 (m, 2H), 3.36 (t, J = 7.7, 1H), 4.04 (m, 2H), 7.23 (m, 5H), ¹³C NMR (62.9 MHz; CDCl₃) δ 12.24, 14.22, 26.88, 53.63, 60.66, 127.16, 128.02, 128.58, 139.34, 174.14; IR (KBr)/cm⁻¹: 3070w, 3050w, 3015s, 2970vs, 2920s, 2880w, 2860w, 1735vs, 1595w, 1580s, 1450vs, 1160br, 740s, 680s.

R-(-)-2-Phenylbuttersäureethylester R-(-)-6: 120 µl R-(-)-12 (126.6 mg, 0.77 mmol) erbrachten 80 mg R-(-)-6 (0.42 mmol, 55 %); [α]_{D}²² = -65.7 (c1.508, Et₂O).

(±)-2-Phenylpropionsäureethylester (±)-7: 2.0 g (±)-13 (13.3 mmol) erbrachten 1.1 g (±)-7 (6.2 mmol, 47 %) als farbloses Öl: Analysen Ergebnis: C, 74.08; H, 8.01; kalk. für C₁₁H₁₄O₂: C, 74.13; H, 7.92; ¹H NMR (250.1 MHz; CDCl₃) δ 1.21 (t, J = 7.1, 3H), 1.51 (d, J = 7.2, 3H), 3.72 (q, J = 7.2, 1H), 4.13 (m, 2H), 7.23-7.34 (m, 5H); ¹³C NMR (62.9 MHz; CDCl₃; Me₄Si) δ 14.19, 18.68, 45.64, 60.79, 127.13, 127.54, 128.65, 140.78, 174.63; IR (KBr)/cm⁻¹: 3070w, 3050w, 3015s, 2970vs, 2920vs, 2880w, 2860w, 1760vs, 1595w, 1445s, 1160br, 840s, 750s, 720s, 680s.

R-(-)-2-Phenylpropionsäureethylester R-(-)-7: 60 µl R-(-)-13 (65.8 mg, 0.44 mmol) erbrachten 38 mg R-(-)-7 (0.21 mmol, 48 %); [α]_{D}²² = -60.0 (c1.630, CHCl₃).

(±)-3-Phenylbuttersäureethylester (±)-8: 5.0 g (±)-14 (30.5 mmol) erbrachten 3.1 g (±)-8 (16.1 mmol, 53 %) als farbloses Öl: Analysen Ergebnis: C, 74.89; H, 8.52; kalk. für C₁₂H₁₆O₂: C, 74.97; H, 8.39; ¹H NMR (250.1 MHz; CDCl₃) δ 1.19 (t, J = 7.1; 3H), 1.32 (d, J = 7.0, 3H), 2.58 (m, 2H), 3.30 (m, 1H), 4.09 (q, J = 7.1, 2H), 7.17-.7.36 (m, 5H); ¹³C NMR (62.9 MHz; CDCl₃) δ 14.24, 21.88, 36.60, 43.08, 60.31, 126.45, 126.84, 128.54, 145,83, 172.46; IR (KBr)/cm⁻¹: 3070w, 3050w, 3020w, 2960vs, 2920s, 2860w, 1735vs, 1595w, 1445s, 1160br, 740s, 680s.

S-(+)-3-Phenylbuttersäureethylester S-(+)-8: 120 µl S-(+)-14 (128.3 mg, 0.78 mmol) erbrachten 60 mg S-(+)-8 (0.31 mmol, 40 %); [α]_{D}²² = 21.4 (c1.538, Et₂OH).

### Herstellung der Hexylester und Analysen

Zur Herstellung der Hexylester wurden die entsprechenden Carbonsäuren zusammen mit Toluol-4-sulfonsäure (20 mg) in einem Gemisch aus n-Hexanol (3,5 ml) und Toluol (50 ml) in einem Rundkolben gelöst. Der Kolben wurde mit einem Rückflußkühler und einer Dean-Stark-Falle versehen, bevor die Lösung refluxiert wurde. Die Reaktion wurde solange durchgeführt bis sich kein Wasser mehr abschied. Anschließend wurde das Gemisch mit Eiswasser (30 ml), gesättigter Na₂CO₃-Lösung (30 ml) und erneutem Wasser (30 ml) gewaschen. Nach Trocknung über Na₂SO₄ und abziehen des Lösungsmittel wurde das Produkt über eine Silicagel-Chromatographie (Petrolether: Et₂OH, 40:1) gereinigt. Es wurde ein farbloses Öl erhalten.

(±)-2-Phenylbuttersäurehexylester (±)-9: Die Herstellung erfolgte nach der oben beschriebenen Methode unter Verwendung von 500 mg (= 3.05 mmol) der entsprechenden Carbonsäure [2-Phenylbutersäure = (±)-12]. Es wurden 510 mg (±)-9 (2.05 mmol, 67 %) erhalten. Analysen Ergebnis: C, 77.54; H, 9.78.; kalk. für C₁₆H₂₄O₂: C, 77.38; H, 9.74; ¹H NMR (250.1 MHz; CDCl₃) δ 0.76-0.85 (m, 6H), 1.17 (m, 6H), 1.49 (t, J = 6.7, 2H), 1.66-2.09 (m, 2H), 3.36 (t, J = 7.7, 1H), 3.98 (dt, J = 6.6, 1.8, 2H), 7.16-7.25 (m, 5H); ¹³C NMR (62.9 MHz; CDCl₃) δ 12.25, 14.02, 22.53, 25.52, 26.73, 28.59, 31.40, 53.70, 64.82, 127.15, 128.02, 128.56, 139.36, 141.32, 174.20; IR (KBr)/cm⁻¹: 3070w, 3050w, 3015w, 2950vs, 2920vs, 2860s, 2840s, 1730vs, 1595w, 1485s, 1447s, 1160, 890w, 750w, 720s, 680vs.

R-(-)-2-Phenylbuttersäurehexylester R-(-)-9: 120 µl R-(-)-12 (126.6 mg, 0.77 mmol) erbrachten 61 mg R-(-)-9 (0.25 mmol, 32 %); [α]_{D}²² = -29.3 (cl.438, CHCl₃).

(±)-2-Phenylpropionsäurehexylester (±)-10: 100 µl (±)-13 (109.7 mg, 0.73 mmol) erbrachten 91 mg (±)-10 (0.39 mmol, 53 %): Analysen Ergebnis: C, 76.93; H, 9.56; kalk. für C₁₅H₂₂O₂: C, 76.88; H, 9.46; ¹H NMR (250.1 MHz; CDCl₃) δ 0.78 (t, J = 6.6, 3H), 1.14-1.22 (m, 6H); 1.42 (d, J = 7.2, 3H), 1.48 (t, J = 6.8, 2H) (q, J = 7.2, 1H); 3.97 (t, J = 6.7, 2H), 7.15-7.25 (m, 5H); ¹³C NMR (62.9 MHz; CDCl₃) 14.00, 21.88, 22.52, 25.54, 28.55, 31.41, 36.56, 43.01, 64.50, 126.37, 126.75, 128.47, 145.75, 172.60; IR (Kbr)/cm⁻¹: 3075w, 3050w, 3015w, 2940vs, 2920vs, 2860s, 2840s, 1735vs, 1595w, 1485w, 1445vs, 1160vs, 890w, 750w, 710w, 680vs.

R-(-)-2-Phenylpropionsäurehexylester R-(-)-10: 60 µl R-(-)-12 (65.8 mg, 0.44 mmol) erbrachten 39 mg R-(-)-10 (0.17 mmol, 39 %); [α]_{D}²² = -35.8 (c1.580, CHCl₃).

(±)-3-Phenylbuttersäurehexylester (±)-11: 130 mg (±)-14 (0.79 mmol) erbrachten 98 mg (±)-11 (0.39 mmol, 49 %): Analysen Ergebnis: C, 77.42; H, 9.80; kalk. für C₁₆H₂₄O₂: C, 77.38; H, 9.74; ¹H NMR (250.1 MHz; CDCl₃) δ 0.81 (t, J = 6.7, 3H), 1.18-1.26 (m, 9H), 1.46 (t, J = 6.8, 2H), 2.51 (m, 2H), 5.80 (m, 1H), 3.93 (t, J = 6.7, 2H), 7.11-7.25 (m, 5H); ¹³C NMR (62.9 MHz; CDCl₃) δ 14.00, 21.88, 22.52, 25.54, 28.55, 31.41, 36.56, 43.01, 64.50, 126.37, 126.75, 128.47, 145.75, 172.50; IR (KBr)/cm⁻¹: 3070w, 3050w, 3015w, 2950vs, 2920vs, 2860s, 2840s, 1740vs, 1595w, 1485s, 1445vs, 1160vs, 890w, 745s, 710s, 680vs.

R-(-)-3-Phenylbuttersäurehexylester R-(-)-11: 120 µl R-(-)-14 (128.3 mg, 0.78 mmol) erbrachten 78 mg R-(-)-11 (0.31 mmol, 40 %); [α]_{D}²² = -21.6 (c2.730, CHCl₃).

Lipase-katalysierte Umesterung: 100 µl der Vinylester (0.52 mmol) und 560 µl n-Hexanol (4,49 mmol) wurden in 5 ml Toluol in einem 10 ml Rundkolben gelöst und das Reaktionsgemisch auf einem thermostatisierten Magnetrührer bei 40 °C oder 60 °C gemischt. Die Reaktion wurde durch Zugabe von 100 mg Lipase (500 U) CAL-B gestartet (siehe Schema II). Proben wurden der Reaktion entnommen, mit Toluol verdünnt und die Lipase wurde über Zentrifugation entfernt. Die Proben wurden mit der GC unter Verwendung der Optima 5-Säule analysiert. Nach Beendigung der Reaktion durch Filtration, wurde Produkt und nicht-umgesetztes Substrat über eine Flash-Chromatographie gereinigt. Die Enantiomerenreinheiten wurden über den Drehwert bestimmt. Im Falle der Substrate (±)-3, (±)-4 und (±)-6 wurde die optische Reinheit außerdem über eine chirale GC bestimmt. Die Ethylester wurden als Vergleichsversuch entsprechend umgesetzt (siehe Schema III). Als weiter Vergleichsversuch wurde die Hydrolyse der Ethylester durchgeführt (siehe Schema III).

### Hydrolyse der Ethylester

Die Hydrolyse der Ethylester erfolgte unter pH-Statisierung. Im allgemeinen wurde 1 mmol Substrat (6-8) in 20 ml Phosphatpuffer (50 mM, pH 7,5) bei 40 °C aufgenommen. 200 mg CAL-B (1000 U) wurden zum Start der Reaktion zugesetzt. Der pH wurde mit 0.1 N NaOH über die Reaktion konstant gehalten. Nachdem der Basenverbrauch eine deutlichte Umsetzung des Substrats anzeigte, wurde nicht-verbrauchtes Substrat über eine Extraktion mit Heptan entfernt. Anschließend wurde der wäßrige Überstand mit NaCl gesättigt und der pH auf pH 3 mit H₂SO₄ eingestellt und anschließend 3 mal mit EtOAc extrahiert, um die freie Carbonsäure (12-14) zu erhalten. Die organischen Phasen wurden mit Na₂SO₄ getrocknet und der Enantiomerenüberschuß wurde für alle Verbindungen über Drehwert und für (±)-6 außerdem über chirale GC bestimmt.

Die Ergebnisse dieser Umesterungs- und Hydrolyseversuche sind Tabelle I zu entnehmen.

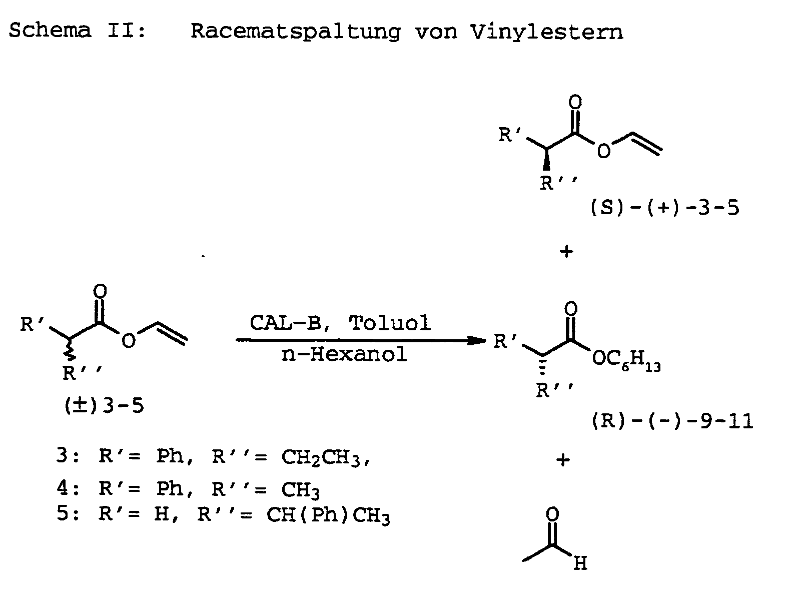

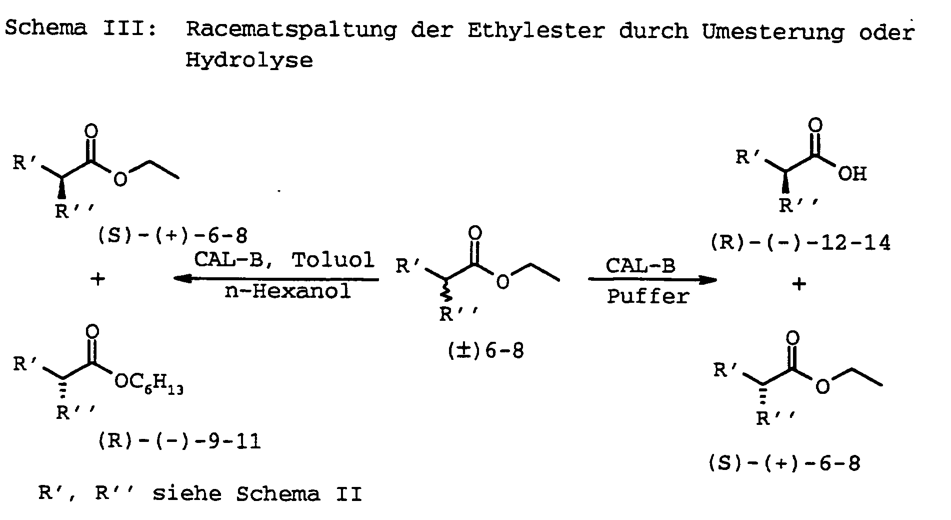

**Tabelle I: Ergebnisse der Umesterung mit Vinylestern oder Ethylestern und der Hydrolyse mit Ethylestern**

| Verbindung | Reaktionszeit^{a} (h) | Ausbeute (%) | Enantiomerenüberschuß | | E^{b} |
|---|---|---|---|---|---|
| | | | (%eeₛ)^{c} | (%eeₚ)^{d} | |
| (±)-3 | 68 | 43 | 74(3) | 99(9) | >100 |
| (±)-6 | 304 | 18 | 14(6) | 66(9) | 5.7(110)^{e} |
| (±)-6 | 1 | 43 | 34(6) | 22(12) | 3.7 |
| (±)-4 | 1.3 | 47 | 75(4) | 53(10) | 26 |
| (±)-7 | 44 | 44 | 41(7) | 68(10) | 6.8(123)^{e} |
| (±)-7 | 0.3 | 47 | 38(7) | 28(13) | 3.5 |
| (±)-5 | 68 | 56 | 86(5) | 31(11) | 13 (23^{f}) |
| (±)-8 | 720 | 19 | 22(8) | 60(11) | 4.9(135)^{e} |
| (±)-8 | 3 | 56 | 93 (8) | 13(14) | 9 |

| | | | | | |
|---|---|---|---|---|---|
| ^{a} Reaktion bei 40 °C ^{b} Kalkuliert nach Chen et al., J. Am. Chem. Soc., 104, 1982: 7294 ^{c} Alles nicht umgesetzte Substrat hatte S-Konfiguration ^{d} Alles hergestellte Produkt hatte R-Konfiguration ^{e} Die Werte in den Klammern entsprechen der Gleichgewichtskonstanten kalkuliert entsprechend Anthonsen et al., Tetrahedron Asymmetry 7, 1996: 2633 ^{f} bei Raumtemperatur (ca. 23 °C) | | | | | |

### Herstellung von Ibuprofenvinylester und Analysen

### Chemische Synthese von Ibuprofenvinylester

In 48 ml Vinylacetat (44,7 g, 0.52 mol) wurden 1g Ibuprofen (4,85 mmol), 171 mg Palladium(II)acetat (0,76 mmol) und 27 mg Kaliumhydroxid (0,48 mmol) gelöst. Die Lösung wurde 18 Std. unter Rückfluss erhitzt. Zur Aufarbeitung wurde Feststoff aus der Reaktionslösung abfiltriert, das Vinylacetat ab destilliert und das zurückbleibende Rohprodukt mittels Blitz-Chromatographie über Kieselgel (Laufmittel: Petrolether/Diethylether 20:1) gereinigt. Ausbeute: 0,92 g (3,96 mmol, 82 %).

### Lipase-katalysierte Umesterung mit Methanol

Der racemische Ibuprofenvinylester (209 mg, 0,90 mmol) und Methanol (240 mg, 7.5 mmol) wurden in 8 ml Toluol gelöst und bei 40 °C gerührt. Die Reaktion wurde durch Zugabe von 416 mg Lipase CAL-B (L-2, c.f., C2, lyo.; Boehringer Mannheim) gestartet. Nach 5 h wurde die Reaktion durch abfiltrieren der Lipase beendet und das Reaktionsgemisch mittels Gaschromatographie an einer chiralen Säule (2,3 Di-*O*-methyl-6-*O*-pentyl-â-cyclodextrin) analysiert. Produkt und nicht umgesetztes Substrat wurden mittels Blitz-Chromatographie über Kieselgel (Laufmittel: Petrolether / Diethylether 35:1) gereinigt.
Umsatz (GC): 40,5 %;
Ausbeuten: Ibuprofenmethylester 64 mg (0,29 mmol; 32 %),
eeₚ = 76 % (GC); Ibuprofenvinylester 98 mg (0,42 mmol, 47 %),
eeₛ = 52 % (berechnet aus Umsatz und eeₚ). Aus Umsatz und eep berechnet sich ein E-Wert von 12.

Ibuprofenmethylester: ¹H-NMR (250,1 MHz, CDCl₃), δ [ppm]: 0,82 (d, 6H, *J* = 6,6 Hz); 1,41 Hz (d, 3H, *J* = 7,2 Hz); 1,77 (septett, 1H, *J* = 6,8); 2,37 (d, 2H , *J* = 7,2 Hz); 3,58 (s, 3 H); 3,70 (q, 1 H, *J =* 7,2 Hz); 7,00-7,18 (m, 4 H). ¹³C-NMR (62,9 MHz, CDCl₃), δ [ppm]: 18,70; 22,47; 30,25; 45,10; 45,11; 52,05; 127,20; 129,43; 137,82; 140,63; 175,30

Ibuprofenvinylester: ¹H-NMR (250,1 MHz, CDCl₃), δ [ppm]: 0,90 (d, 6H, *J* = 6,6 Hz); 1,52 Hz (d, 3H, *J* = 7,1 Hz); 1,85 (septett, 1H, *J =* 6,7); 2,45 (d, 2H , *J* = 7,2 Hz); 3,75 (q, 1 H, *J* = 7,2 Hz); 4,55 (dd, 1H, *J* = 1,6, *J* = 6,3); 4,86 (dd, 1H, *J =* 1,6, *J =* 14,0); 7,00-7,18 (m, 4 H). ¹³C-NMR (62,9 MHz, CDCl₃), δ [ppm]: 18,50; 22,46; 30,25; 44,96; 45,11; 97,92; 127,27; 129,51; 136,97; 140,89; 141,50; 171,90

## Patentansprüche

1. Verfahren zur Racematspaltung von Arylalkylcarbonsäureestern der allgemeinen Formel I 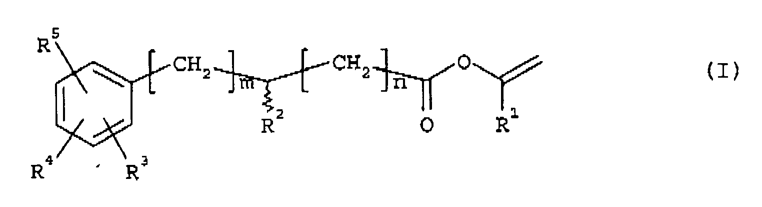 **dadurch gekennzeichnet, daß** man Verbindungen der allgemeinen Formel I in Gegenwart einer Lipase oder Esterase mit einem Alkohol der allgemeinen Formel R⁶-OH zu Verbindungen der allgemeinen Formeln Ia und II 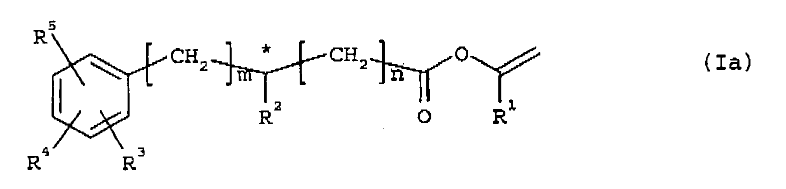 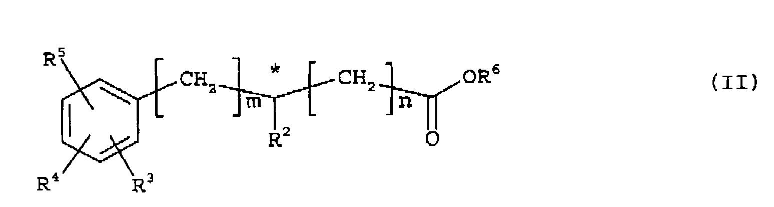 umsetzt,
wobei mindestens eine der Verbindungen der Formeln Ia oder II in einem Enantiomerenüberschuß vorliegt und die Substituenten und Variablen in den Formeln I, Ia und II folgende Bedeutung haben:
* = optisch aktives Zentrum
n und m unabhängig voneinander 0 oder 1
R¹ = Wasserstoff oder Methyl
R² = substituiertes oder unsubstituiertes, verzweigtes oder unverzweigtes C₁-C₆-Alkyl-, C₃-C₆-Cycloalkyl-, Aryl- oder Hetaryl-,
R³, R⁴, R⁵ unabhängig voneinander Wasserstoff, substituiertes oder unsubstituiertes, verzweigtes oder unverzweigtes C₁-C₁₀-Alkyl-, C₂-C₁₀-Alkenyl-, C₃-C₁₀-Alkinyl-, C₁-C₁₀-Alkoxy-, C₂-C₁₀-Alkenyloxy-, C₂-C₁₀-Alkinyloxy-, C₃-C₁₀-Cycloalkyl-, C₃-C₁₀-Cycloalkyloxy-, C₁-C₄-Alkylaryl-, C₁-C₄ -Alkylhetaryl-, Aryl-, Hetaryl-, Hydroxyl-, Halogen-, Cyano-, Nitro- oder Amino-,
R⁶ = substituiertes oder unsubstituiertes, verzweigtes oder unverzweigtes C₁-C₂₀-Alkyl-, C₁-C₂₀-Alkoxy-, C₂-C₂₀-Alkenyl- oder C₃-C₁₀-Cycloalkyl-
und wobei zwei benachbarte Substituenten R³, R⁴ oder R⁵ zusammen einen weiteren substituierten oder unsubstituierten aromatischen, gesättigten oder teilweise gesättigten Ring mit 5 bis 6 Atomen im Ring bilden können, der ein oder mehrere Heteroatome wie O, N oder S enthalten kann.

2. Verfahren nach Anspruch 1, **dadurch gekennzeichnet, daß** die Racematspaltung in Gegenwart eines weiteren organischen Lösungsmittels durchgeführt wird.

3. Verfahren nach Anspruch 1 oder 2, **dadurch gekennzeichnet, daß** die Racematspaltung bei Temperaturen zwischen -50 °C und +100 °C durchgeführt wird.

4. Verfahren nach den Ansprüchen 1 bis 3, **dadurch gekennzeichnet, daß** bakterielle, pilzliche Lipasen oder Schweinepankreaslipase verwendet werden.

5. Verfahren nach den Ansprüchen 1 bis 4, **dadurch gekennzeichnet, daß** eine bakterielle oder pilzliche Lipase der Gattungen Arthrobacter, Bacillus, Brevibacterium, Pseudomonas, Chromobacterium, Aspergillus, Candida, Fusarium, Geotrichum, Humicola, Mucor, Pichia oder Rhizopus verwendet wird.

6. Verfahren nach den Ansprüchen 1 bis 5, **dadurch gekennzeichnet, daß** als Lipase Candida antarctica Lipase verwendet wird.

## Claims

1. A process for the resolution of esters of arylalkylcarboxylic acids of the general formula I 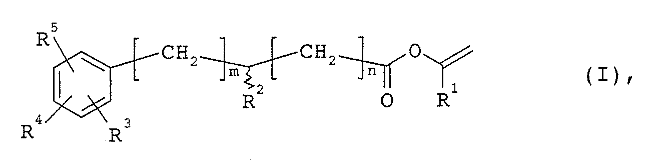 which comprises reacting compounds of the general formula I with an alcohol of the general formula R⁶-OH in the presence of a lipase or esterase to give compounds of the general formulae Ia and II 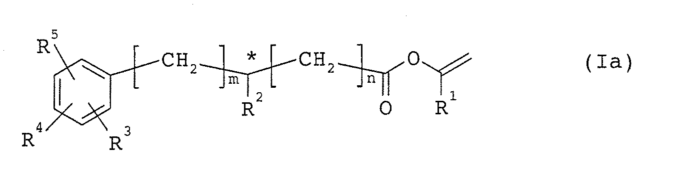 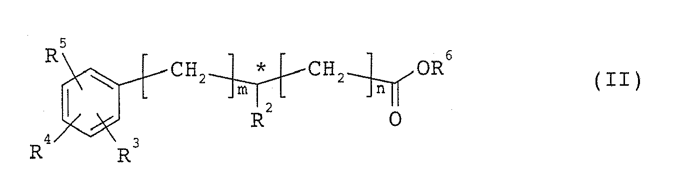 at least one of the compounds of the formulae Ia or II being present in an enantiomeric excess and the substituents and variables in the formulae I, Ia and II having the following meanings:
* = optically active center
n and m independently of one another are 0 or 1
R¹ = hydrogen or methyl
R² = substituted or unsubstituted, branched or unbranched C₁-C₆-alkyl-, C₃-C₆-cycloalkyl-, aryl- or hetaryl-,
R³, R⁴, R⁵ independently of one another are hydrogen, substituted or unsubstituted, branched or unbranched C₁-C₁₀-alkyl-, C₂-C₁₀-alkenyl-, C₂-C₁₀-alkynyl-, C₁-C₁₀-alkoxy-, C₂-C₁₀-alkenyloxy-, C₂-C₁₀-alkynyloxy-, C₃-C₁₀-cycloalkyl-, C₃-C₁₀-cycloalkyloxy-, C₁-C₄-alkylaryl-, C₁-C₄-alkylhetaryl-, aryl-, hetaryl-, hydroxyl-, halogen-, cyano-, nitro- or amino-,
R⁶ = substituted or unsubstituted, branched or unbranched C₁-C₂₀-alkyl-, C₁-C₂₀-alkoxy-, C₂-C₂₀-alkenyl-, C₃-C₁₀-cycloalkyl-
and where two adjacent substituents R³, R⁴ or R⁵ together can form a further substituted or unsubstituted aromatic, saturated or partially saturated ring having 5 to 6 atoms in the ring, which can comprise one or more heteroatoms such as O, N or S.

2. The process according to claim 1, wherein the resolution is carried out in the presence of a further organic solvent.

3. The process according to claim 1 or 2, wherein the resolution is carried out at temperatures between -50°C and +100°C.

4. The process according to any of claims 1 to 3, wherein bacterial, fungal lipases or porcine pancreatic lipase are/is used.

5. The process according to any of claims 1 to 4, wherein a bacterial or fungal lipase of the genera Arthrobacter, Bacillus, Brevibacterium, Pseudomonas, Chromobacterium, Aspergillus, Candida, Fusarium, Geotrichum, Humicola, Mucor, Pichia or Rhizopus is used.

6. The process according to any of claims 1 to 5, wherein the lipase used is Candida antarctica lipase.

## Revendications

1. Procédé de dédoublement de mélange racémique d'esters d'acides arylalkylcarboxyliques de la formule générale 1 : 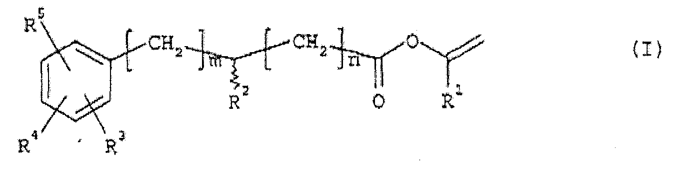 **caractérisé en ce que**, en présence d'une lipase ou d'une estérase, on fait réagir des composés de la formule générale I avec un alcool de la formule générale R⁶-OH pour former des composés des formules générales Ia et II : 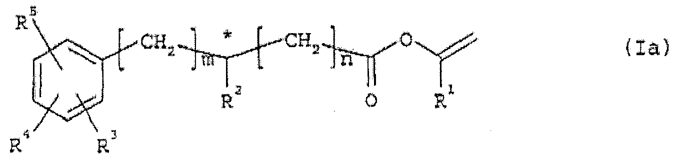 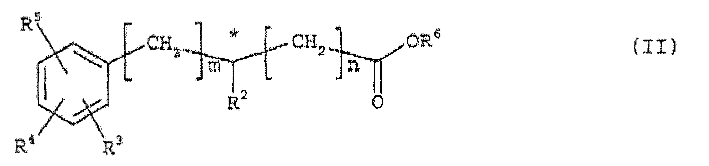 au moins un des composés des formules Ia ou II se présentant dans un excès d'énantiomères, les substituants et variables dans les formules I, Ia et II ayant les significations suivantes :
* = centre optiquement actif,
n et m valent indépendamment l'un de l'autre 0 ou 1,
R¹ = hydrogène ou méthyle,
R² = un groupe substitué ou non substitué, ramifié ou non ramifié, alkyle en C₁-C_{6,} cycloalkyle en C₃-C₆, aryle ou hétaryle,
R³, R⁴, R⁵ = indépendamment l'un de l'autre de l'hydrogène ou un groupe substitué ou non substitué, ramifié ou non ramifié, alkyle en C₁-C₁₀, alcényle en C₂-C₁₀, alcynyle en C₂-C₁₀, alcoxy en C₁-C₁₀, alcényloxy en C₂-C₁₀, alcynyloxy en C₂-C₁₀, cycloalkyle en C₃-C₁₀, cycloalkyloxy en C₃-C₁₀, C₁-C₄-alkylaryle, C₁-C₄-alkylhétaryle, aryle, hétaryle, hydroxyle, cyano, nitro ou amino ou de l'halogène,
R⁶ = un groupe substitué ou non substitué, ramifié ou non ramifié, alkyle en C₁-C₂₀, alcoxy en C₁-C₂₀, alcényle en C₂-C₂₀ ou cycloalkyle en C₃-C₁₀,
deux substituants voisins R³, R⁴ ou R⁵ pouvant former conjointement un autre noyau aromatique, saturé ou partiellement saturé, substitué ou non substitué, comportant 5 à 6 atomes dans le noyau qui peut contenir un ou plusieurs hétéroatomes, tels que O, N ou S.

2. Procédé suivant la revendication 1, **caractérisé en ce que** le dédoublement de racémate est effectué en présence d'un autre solvant organique.

3. Procédé suivant la revendication 1 ou 2, **caractérisé en ce que** le dédoublement de racémate est effectué à des températures comprises entre -50°C et +100°C.

4. Procédé suivant les revendications 1 à 3, **caractérisé en ce qu'**on utilise des lipases bactériennes, des lipases fongiques ou une lipase de pancréas de porc.

5. Procédé suivant les revendications 1 à 4, **caractérisé en ce qu'**on utilise une lipase bactérienne ou fongique des genres Arthrobacter, Bacillus, Brevibacterium, Pseudomonas, Chromobacterium, Aspergillus, Candida, Fusarium, Geotrichum, Humicola, Mucor, Pichia ou Rhizopus.

6. Procédé suivant les revendications 1 à 5, **caractérisé en ce que**, comme lipase, on utilise une lipase de Candida antarctica.
